# EUROPEAN PATENT SPECIFICATION

(11) **EP 0 452 391 B1**
(45) Date of publication and mention of the grant of the patent: **16.07.1997**
(21) Application number: 90901711.3
(22) Date of filing: 02.01.1990
(51) Int. Cl.: C12N 15/19, C07K 14/52, C12P 21/02

(54) **A POLYPEPTIDE HAVING HUMAN MONOCYTE CHEMOTACTIC FACTOR ACTIVITY AND A DNA ENCODING SAID POLYPEPTIDE**
POLYPEPTID MIT EINER AKTIVITÄT DES MENSCHLICHEN MONOCYTEN CHEMOTAKTISCHEN FAKTORS UND DNA, KODIEREND FÜR DAS POLYPEPTID
POLYPEPTIDE POSSEDANT UNE ACTIVITE DE FACTEUR CHIMIOTACTIQUE MONOCYTE HUMAIN ET ADN CODANT CE POLYPEPTIDE

(30) Priority: 01.01.1989 JP 65/89; 03.02.1989 JP 126438/89
(43) Date of publication of application: 23.10.1991
(73) Proprietor: THE UNITED STATES OF AMERICA as represented by the Secretary UNITED STATES DEPARTMENT OF COMMERCE, Washington, DC 20231 (US)
(72) Inventor: FURUTANI, Yasuji, Kobe-shi (JP); FUKUI, Toshikazu, Osaka-fu (JP); JUNICHI, Yamagishi, Nara-shi (JP); MASAAKI, Yamada, Kyoto-shi (JP); MATSUSHIMA, Kouji, Toyamo 930-11 (JP); OPPENHEIM, Joost, Bethesda, MD 20892 (US)
(74) Representative: Jump, Timothy John Simon
(86) International application number: US9000040
(87) International publication number: WO9007863

(56) References cited:
- JOURNAL OF IMMUNOLOGY. vol. 110, no. 3, March 1973, WILIAMS AND WILKINS, BALTIMORE, US pages 801 - 810; L.C. ALTMANN ET AL.: 'A human mononuclear leukocyte chemotactic factor: characterisation, specificity and kinetics of production by homologous leukocytes'
- JOURNAL OF IMMUNOLOGY. vol. 117, no. 1, November 1976, WILIAMS AND WILKINS, BALTIMORE, US pages 1545 - 1552; K.A.FOON ET AL.: 'Serotonin-induced production of a monocyte chemotactic factor by human peripheral blood leukocytes'
- PATENT ABSTRACTS OF JAPAN vol. 10, no. 71 (C-334)(2128) 20 March 1986
- BLOOD , vol. 69, no. 1, January 1987, SAUNDERS, NEW YORK, US; pages 219 - 223; M. PONCZ ET AL.: 'Cloning and characterization of platelet factor 4 cDNA derived from a human erythroleukemic cell line'
- Biochemical & Biophysical Research Comm., 28 February 1989, v. 159(1) pp. 249- 255. FURUTANI. See fig. 2.
- Science, 29 September 1989, v. 245, pp. 1490-1493. Graves, See figs. 2 and 3.
- Journal of Immunology, 1 April 1987, p. 2372-2379. Benomar. See fig. 4.
- Biochemistry 27 (1988) 4162
- FEBS Lett 244 (1989) 487
- PNAS 89 (1992) 5371
- PNAS 85 (1988) 3738
- PNAS 83 (1986) 3316
- J Exp Med 169 (1989) 1449

## Description

This invention relates to a DNA encoding a polypeptide with human monocyte chemotactic factor activity, a polypeptide produced by a transformant cell transformed with an expression vector in which said DNA is inserted, and a process for production of said polypeptide by using said transformant.

Human monocyte chemotactic factor (abbreviated MCF hereinafter) is a physiologically active polypeptide which is produced from human monocytic cells stimulated with lipopolysaccharide (LPS), and has biological activity to attract monocytes or to augment the inhibitory effect of monocytes on tumor cell proliferation. By attracting monocytes and further augmenting monocyte activities, MCF is expected as a drug for treatment of certain bacterial infectious diseases or cancers.

Rollins et al (Proc Natl Acad Sci USA 85 (1988) 3738-42) disclosed the cloning and expression of JE, a murine gene whose product has cytokine-like properties. The chemotactic activity of this cytokine-like protein was not disclosed.

Valente et al (Biochemistry 27 (1988) 4162-68) disclosed the purification of a monocyte chemotactic factor secreted by baboon vascular cells in culture. A sequence for the protein was not disclosed.

Drs. Kouji Matsushima and Joost J. Oppenheim et al. of the present inventors have isolated so-called natural human MCF from the culture media of human monocytic leukemia cells stimulated with some inducers, and determined its partial amino acid sequence. Its molecular weight was estimated to be approximately 15 kDa (J. Exp. Med. 169 : 1482 - 1490, 1989).

The present inventors have succeeded in isolation of a cDNA encoding human MCF by depending upon the defined partial amino acid sequence of the natural human MCF. A human MCF polypeptide was found to be a polypeptide with a lower molecular weight of approximately 9 kDa, consisting of the C-terminal 76 amino acids of its precursor, since the complete primary structure of its precursor polypeptide was established by analyzing the nucleotide sequence of the cloned human MCF cDNA.

The present inventors attempted to express directly a polypeptide having human MCF activity by applying recombinant DNA technology using a transformant cell transformed with an expression vector in which the above cloned DNA or its principal portion is inserted. Consequently, it has been found that said polypeptide could be produced.

Accordingly, in a first aspect of the invention, there is provided an isolated DNA sequence coding for the expression of a polypeptide with human monocyte chemotactic factor activity comprising amino acid sequence [I] or any portion of said amino acid sequence capable of exhibiting human monocyte chemotactic factor activity 
Gln Pro Asp Ala Ile Asn Ala Pro Val Thr
Cys Cys Tyr Asn Phe Thr Asn Arg Lys Ile
Ser Val Gln Arg Leu Ala Ser Tyr Arg Arg
Ile Thr Ser Ser Lys Cys Pro Lys Glu Ala
Val Ile Phe Lys Thr Ile Val Ala Lys Glu
Ile Cys X Asp Pro Lys Gln Lys Trp Val
Gln Asp Ser Met Asp His Leu Asp Lys Gln
Thr Gln Thr Pro Lys Thr [I]
 (wherein X means Ala or Thr).

In a second aspect of the invention, there is provided an isolated DNA sequence deleted 23 to 29 codons from the 5'-terminus of a DNA coding for the expression of a human monocyte chemotactic factor precursor comprising amino acid sequence [II] or its allelic mutant DNA 
Met Lys Val Ser Ala Ala Leu Leu Cys Leu
Leu Leu Ile Ala Ala Thr Phe Ile Pro Gln
Gly Leu Ala Gln Pro Asp Ala Ile Asn Ala
Pro Val Thr Cys Cys Tyr Asn Phe Thr Asn
Arg Lys Ile Ser Val Gln Arg Leu Ala Ser
Tyr Arg Arg Ile Thr Ser Ser Lys Cys Pro
Lys Glu Ala Val Ile Phe Lys Thr Ile Val
Ala Lys Glu Ile Cys X Asp Pro Lys Gln
Lys Trp Val Gln Asp Ser Met Asp His Leu
Asp Lys Gln Thr Gln Thr Pro Lys Thr [II]
 (wherein X means Ala or Thr).

In a third aspect of the invention, there is provided a vector containing a DNA sequence according to the invention in either of its first or second aspects.

In a fourth aspect of the invention, there is provided a microbial host transformed by a vector according to the invention in its third aspect.

In a fifth aspect of the invention, there is provided a process for producing a recombinant polypeptide with human monocyte chemotactic factor activity comprising culturing a transformed microbial host according to the invention in its fifth aspect under conditions suitable for expression of a polypeptide with human monocyte chemotactic factor activity and recovering said polypeptide.

In a sixth aspect of the invention, there is provided a purified polypeptide with human monocyte chemotactic factor activity, obtainable by a process according to the invention in its fifth aspect.

In a seventh aspect of the invention, there is provided an essentially pure polypeptide with human monocyte chemotactic factor activity consisting of amino acid sequence [I] or any portion of said amino acid sequence capable of exhibiting human monocyte chemotactic factor activity.

Further preferred embodiments of the invention in any of its various aspects are as defined in the subclaims.

According to this invention, a polypeptide with human MCF activity consisting of an amino acid sequence represented by the formula [I] or its principal portion (hereinafter referred to as the "polypeptide of this invention"), can be produced by applying recombinant DNA technology using a DNA encoding a polypeptide with human MCF activity consisting of an amino acid sequence represented by the formula [I] or its principle portion.
(referred to as the "DNA of this invention", hereinafter).

Among the DNA of this invention, as a nucleotide sequence of a DNA encoding a polypeptide consisting of an amino acid sequence represented by the formula [I], the DNA consisting of a nucleotide sequence represented by the following formula [A] (to be sometimes abbreviated as the "DNA encoding human MCF") is illustrated. 
CAGCCAGATGCAATCAATGCCCCAGTCACC
TGCTGYTATAACTTCACCAATAGGAAGATC
TCAGTGCAGAGGCTCGCGAGCTATAGAAGA
ATCACCAGCAGCAAGTGTCCCAAAGAAGCT
GTGATCTTCAAGACCATTGTGGCCAAGGAG
ATCTGTRCTGACCCCAAGCAGAAGTGGGTT
CAGGATTCCATGGACCACCTGGACAAGCAA
ACCCAAACTCCGAAGACT
formula [A]
 (wherein Y means C or T, and R means G or A.)

The DNA encoding a human MCF polypeptide can be isolated, for example, according to the method as described in Example 1 and its modified method. It is also possible to perform the total synthesis of said DNA chemically. A DNA encoding the principal portion of human MCF can be produced by the methods of the cleavage and/or repairment of the extra region or deficient region of the DNA encoding human MCF such as, for example, digesting by an appropriate restriction enzyme and ligating with chemically synthesized oligodeoxyribonucleotide(s) and by the technique of site-directed mutagenesis (for example, Kunkel, T.A. et al., Methods in Enzymol., 154, 367-382, 1987).

An expression vector for production of the polypeptide of this invention is constructed according to the technique of gene engineering and the principles of gene expression (for example, Maniatis, T. et al., Molecular Cloning: A Laboratory Manual. Cold Spring Harbor Laboratory, 1982) by adding a translation initiation codon ATG to the 5'-terminus (upstream) of the DNA of this invention, ligating a DNA fragment containing a termination codon to the 3'-terminus (downstream) of the DNA having the initiation codon, connecting the resulting DNA with a proper promoter (e.g. trp, lac, phoS, PL, SV40 early promoter) and SD sequence, and then inserting the resulting DNA into a proper vector (e.g. plasmid pBR322).

A nucleotide sequence from SD sequence to the translation initiation codon is preferably illustrated by formula [B]. 5'-X'GGAGGTTTY'ATT-3'    formula [B] wherein X' means (A)x, x being 1 to 5, and Y' means (A)y(T)z, y being 0 to 3, z being 0 or 1.

A transformant of this invention can be obtained by introducing the expression vector constructed as above into a proper host cell, for example E. coli according to the method of Cohen et al. (Cohen, S.N., et al., Proc. Natl. Acad. Sci., USA, 69, 2110, 1972).

The polypeptide of this invention can be produced by cultivating the transformant of this invention under suitable culture conditions. The extract containing said polypeptide can be obtained from the culture after destroying the cells, for example by lysozyme digestion and freeze-thawing, sonication or by using a French press, followed by collection the extract by centrifugation or filtration.

The polypeptide of this invention can be purified from the extract by purification methods characterized by combination of treatment for removing nucleic acids, salting-out, anion exchange chromatography, cation exchange chromatography, ultrafiltration, gel filtration, if necessary dialysis, electrophoresis, affinity chromatography using specific antibodies, and so on.

Then, depending upon the host cell used and other conditions, a polypeptide with Met residue due to the translation initiation codon at its N-terminus can be produced. It should be understood that such a polypeptide is included within the polypeptide of this invention as long as it has human MCF activity.

The polypeptide of this invention can be also produced by applying a cell-free transcription-translation system using said expression vector.

The polypeptide of this invention means the polypeptide consisting of an amino acid sequence represented by the aforesaid formula [I] or its principal portion which has a certain activity of MCF activities, for example attracting monocytes or augmenting the inhibitory effect of monocytes on tumor cell proliferation. The polypeptide consisting a principal portion of an amino acid sequence represented by the formula [I] includes, for example a polypeptide consisting of said amino acid sequence in which the N-terminal one to six amino acids or the C-terminal six amino acids are deleted.

It should be understood that a polypeptide encoded in the allelic mutant DNA encoding human MCF and a principal portion of said polypeptide are included within the polypeptide of this invention.

Furthermore, it should be understood that a polypeptide resulting from the adding to the N-terminus of the polypeptide consisting of an amino acid sequence represented by the formula [I], an amino acid or a peptide which is, for example, corresponding to a part of the C-terminal amino acid sequence of prepeptide region in the human MCF precursor polypeptide is included within the polypeptide of this invention.

The polypeptide of this invention produced by applying recombinant DNA technology is characterized according to the following methods.

The molecular weight was measured by SDS-polyacrylamide gel electrophoretic analysis in comparison of mobility with those of the molecular weight marker proteins (Standard Protein Kit: Pharmacia, Sweden).

Monocyte chemotactic activity was measured in a chemotaxis Boyden chamber (Neuro Probe, Inc., USA). Namely, the polypeptide of this invention was added to the lower chamber and human monocyte was added into the upper chamber. The lower and upper chambers are separated with a 8 micrometers pore size polycarbonate filter (Nucleopore, USA). After incubating the chamber at 37°C, the migrated cells which adhered to the lower surface of the filter, fixed with methanol and stained with Giemsa solution, were counted by microscopic analysis. RPMI-1640 medium supplemented 0.5% bovine serum albumin was used for dilution of the polypeptide of this invention and for incubation.

For formulating the polypeptide of this invention, it is preferred to add a vehicle and a stabilizer to the preparation. Examples of the stabilizer are albumin, globulin, gelatin, protamine, protamine-salts, glucose, galactose, xylose, mannitol, glucuronic acid, treharose, dextran, hydroxyethyl starch, nonionic surface-active agents and so on.

For simplification of the description, the following abbreviations are used in the present specification and claims.
- A:: adenine
- C:: cytosine
- G:: guanine
- T:: thymine
- RNA:: ribonucleic acid
- mRNA:: messenger RNA
- DNA:: deoxyribonucleic acid
- cDNA:: complementary DNA
- sscDNA:: single-stranded cDNA
- dscDNA:: double-stranded cDNA
- ATP:: adenosine triphosphate
- dATP:: deoxyadenosine triphosphate
- dCTP:: deoxycytidine triphosphate
- dGTP:: deoxyguanosine triphosphate
- dTTP:: deoxythymidine triphosphate
- SD sequence:: Shine-Dalgarno sequence
- kb:: kilobase
- kbp:: kilobase pairs
- bp:: base pairs
- LPS:: lipopolysaccharide
- EDTA:: ethylenediaminetetraacetic acid
- DTT:: dithiothreitol
- kDa:: kilodaltons
- SDS:: sodium laurylsulfate
- MOPS:: 3-N-(Morpholino)propanesulfonic acid

The following Examples and Referential Examples illustrate this invention more specifically, however, it should be understood that the invention is in no way limited to these examples.

### EXAMPLE 1

### Cloning of DNA encoding human MCF

Human promyelocytic leukemia cell line, HL-60 cell (ATCC No. CCL-240) were seeded in Petri Dishes (90x16mm) at a cell density of 1x10⁶ cells per ml. RPMI-1640 medium containing 10% fetal bovine serum was used as a culture medium. In the culture medium supplemented with phorbol-12-myristate-13-acetate (PMA) and retinoic acid to final concentrations of 500 ng/ml and 1 microgram/ml, respectively, the cells were cultivated in air containing 5% carbon dioxide at 37°C and a humidity of 90 to 100%, for 2 days. After the pre-cultivation, the conditioned medium and non-adhered cells were removed by suction. The differentiated adhered cells were further cultivated for 6 hours in RPMI-1640 medium containing 10% fetal bovine serum with LPS and cycloheximide to final concentrations of 10 micrograms/ml and 1 microgram/ml, respectively, under the same conditions as above. After the cultivation, the conditioned medium was removed by suction, the cells adherent to the dishes were lysed and homogenized in a 6M guanidyl thiocyanate solution containing 0.5% sodium N-lauroyl sarcosinate, 6mM sodium citrate and 0.1M 2-mercaptoethanol. The homogenate was applied to a 5.7M cesium chloride solution containing 0.1M EDTA, and centrifuged for 20 hours at 26,500 rpm using an ultracentrifuge (RP27-2 rotor, Hitachi Koki, Japan) to obtain a total RNA fraction as a pellet. The pellet was dissolved in a small amount of 7M urea solution containing 0.35M NaCl, 20mM Tris and 20mM EDTA, and the total RNA was recovered by precipitation from ethanol.

The total RNA was dissolved in 10mM Tris-HCl buffer (pH 7.4) containing lmM EDTA, and the solution was heated at 65°C for 5 minutes. A NaCl solution was added to a final concentration of 0.5M, and the solution was applied onto a column of oligo(dT)-cellulose previously equilibrated with 10mM Tris-HCl buffer (pH 7.4) containing lmM EDTA and 0.5M NaCl. The mRNA was isolated from the column by eluting with 10mM Tris-HCl buffer (pH 7.4) containing lmN EDTA.

The mRNA obtained was used as a template for synthesizing cDNA according to the method of Gubler and Hoffman (Gene, 25, 263, 1983). Six micrograms of the mRNA was dissolved in distilled water (6 micrograms/6 microliters), and then added 0.6 microliter of 100mM methylmercuric hydroxide. After standing for 10 minutes at room temperature, 1.8 microliters of 0.5M 2-mercaptoethanol containing about 20 units of RNase inhibitor (RNasin: Promega, USA) to the solution. After standing for 5 minutes at room temperature, 32 microliters of 50mM Tris-HCl buffer (pH 8.3) containing 10nM magnesium chloride, 1.25mN dGTP, 1.25mM dATP, 1.25mM dTTP, 0.5mN dCTP, 170nM α-³²P-dCTP (specific radioactivity, 6,000Ci/mmole), 4 micrograms of oligo(dT)₁₂₋₁₈ and 120 units of reverse transcriptase derived from avian myeloblastosis virus (Bio-Rad Labs., USA) was added to the solution, and then incubated 42°C for 60 minutes. The reaction was stopped by adding 2 microliters of 0.5M EDTA. The resulting product (sscDNA-mRNA hybrid) was extracted with phenol/chloroform (1:1), and recovered by precipitation with ethanol from the aqueous phase added ammonium acetate to a final concentration of 2.5M.

The product (sscDNA-mRNA hybrid) was dissolved in 100 microliters of a second synthesis buffer [composition: 20mM Tris-HCl buffer (pH 7.5) containing 5mM magnesium chloride, 10mM ammonium sulfate, 100mM potassium chloride, 0.15mM β-nicotinamide adenine dinucleotide, 0.04mM dGTP, 0.04mM dATP, 0.04mM dTTP, 0.04mM dCTP, 5 micrograms of bovine serum albumin, 1.25 units of E. coli ribonuclease H and 24 units of E. coli DNA polymerase I]. The reaction mixture was incubated at 12°C for 60 minutes, and added 2.5 units of E. coli DNA ligase and further incubated 22°C for 60 minutes. The reaction was stopped by adding EDTA. The reaction product (dscDNA) was extracted with phenol/chloroform (1:1) and recovered by precipitation from ethanol.

The product (dscDNA) was dissolved in 100 microliters of an oligo(dC) tailing buffer [composition: 100mM sodium cacodylate buffer (pH 7.2) containing 2mM cobalt chloride, 0.2mM DTT, 0.1mM dCTP and 10 units of terminal deoxynucleotidyl transferase], and incubated at 37°C for 30 minutes to permit the addition of oligo(dC) tails to the 3'-termini of dscDNA. The reaction product [oligo(dC)-tailed dscDNA] was extracted with phenol/chloroform (1:1) and recovered by precipitation from ethanol.

The oligo(dC)-tailed dscDNA obtained as above and an oligo(dG)-tailed pBR322, PstI cut (Bethesda Res. Labs., USA) were dissolved and mixed in an annealing buffer [composition: 10mM Tris-HC1 buffer(pH 7.4) containing 1mM EDTA and 100mM NaC1] and incubated at 65°C for 10 minutes, at 57°C for 2 hours and at 45°C for 2 hours to perform annealing the oligo(dC)-tails to the oligo(dG)-tails in order to prepare recombinant double-stranded plasmids.

The recombinant plasmids obtained as above were introduced into E. coli HB101 according to the following method to construct human cDNA library. Namely, E. coli HB101 was inoculated in L broth [composition: 1% tryptone, 0.5% yeast extract, 0.5% NaC1, 0.1% glucose (pH 7.2)], and cultivated at 30°C until the turbidity at 600nm reached 0.5. The culture was allowed to stand in an ice-water for 30 minutes, and then the cells were collected by centrifugation. The cells were resuspended in 50mM calcium chloride and allowed to stand in an ice-water for 60 minutes, and then the cells were collected by centrifugation. The cells were resuspended in 50mM calcium chloride containing 20% glycerin. To the cell suspension was added the recombinant plasmid solution and mixed. The mixture was allowed to stand in an ice-water for 20 minutes and then maintained at room temperature for 10 minutes. Then, L broth was added, and cultivated with shaking at 37°C for 60 minutes. An aliquot of the culture was taken, spread on L broth agar plate (agar concentration: 1.5%) containing 6.25 micrograms/ml of tetracycline, and cultivated at 37°C overnight. A human cDNA library was prepared by selecting transformants resistant to tetracycline.

In order to screen the cDNA library for transformants which had a plasmid containing cDNA encoding human MCF, colony hybridization assay was done according to the method of Hanahan and Meselson (Gene, 10, 63, 1980) using the following chemically synthesized oligodeoxyribonucleotide probes.

Namely, four kinds of oligodeoxyribonucleotides represented by the following formulae [1] to [4] were chemically synthesized based on the defined partial amino acid sequence of so-called natural human MCF that purified from human cell line, THP-1 cells, Met-Asp-His-Leu-Asp-Lys-Gln-Thr-Gln-Thr-Pro-Lys-Thr, and they were used as probes. 5'-ATGGAYCAYTTRGA-3'    [1] 5'-ATGGAYCAYCTNGA-3'    [2] 5'-GAYAARCARACYCA-3'    [3] 5'-GAYAARCARACRCA-3'    [4] wherein Y means C and T, R means G and A, and N means T, C, A and G. Therefore, the probe represented by the formula [1] is a pool of 8 kinds of DNAs (14-mer), and each probe represented by the formulae [2] to [4] is a pool of 16 kinds of DNAs (14-mer).

Each synthesized probe (100 pmole) of the above formulae was end-labelled with ³²p under the reaction conditions using γ-³²P-ATP (approximately 50 pmole: specific radioactivity, 5,000Ci/mmole) and T4 polynucleotide kinase (10 units).

The human cDNA library was screened for the clones containing a cDNA having nucleotide sequence hybridizing with both the following two sets of probes. One set was a mixture of the pool probes of the formulae [1] and [2], and another set was a mixture of the pool probes of the formulae [3] and [4]. Colony hybridization was carried out under the condition of 36°C for 40 hours. As a result, 35 clones were selected from about 36,000 clones. The cDNAs were isolated from the first selected these clones, and subjected to restriction enzyme mapping analysis. It was confirmed by this analysis that all these cDNAs contain the common nucleotide sequence.

Nucleotide sequences of the finally selected three recombinant plasmids (plasmid No. pHMCF7, pHMCF25 and pHMCF29) were determined by the dideoxy chain termination method according to the instruction manual (Takara Shuzo Co., Japan), by using a 7-DEAZA sequencing kit (Takara Shuzo Co.), and pUC18 and pUC19 as a cloning vector.

Nucleotide sequence encoding human MCF precursor and the predicted amino acid sequence from the nucleotide sequence are summarized in Table 1. In the nucleotide sequence encoding human MCF precursor inserted into pHMCF7 and pHMCF29, the bases at the base No. 105 and the base No. 226 shown in Table 1 were T and G, respectively. On the other hand, the bases at the base No. 105 and the base No. 226 in the nucleotide sequence of pHMCF25 were C and A, respectively.

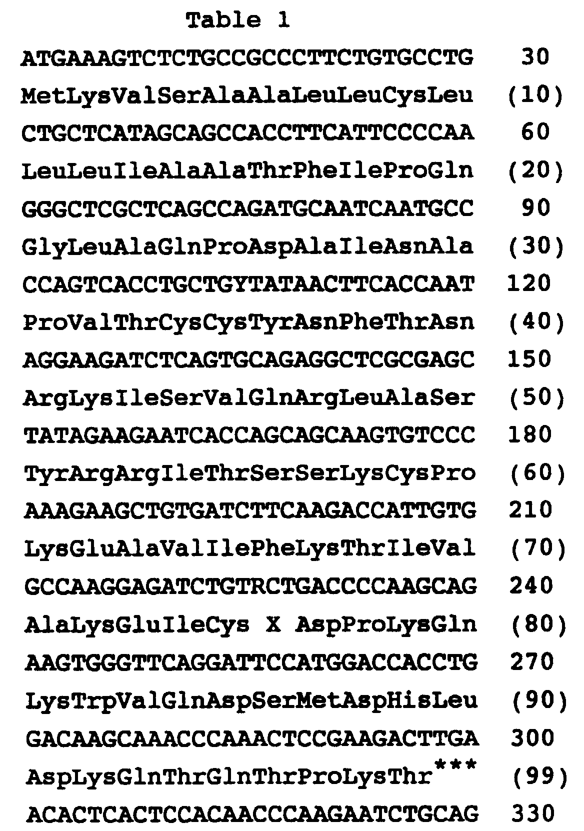

In Table 1, numerals represent the base number. Parenthesized numerals represent the amino acid number. *** means a translation stop codon. A nucleotide sequence from the base No. 1 to the base No. 297 is a nucleotide sequence encoding human MCF precursor, and a nucleotide sequence from the base No. 70 to the base No. 297 (corresponding to the nucleotide sequence represented by the formula [A]) is a nucleotide sequence encoding human MCF; Y at the base No. 105 means C or T, and R at the base No. 226 means G or A. An amino acid sequence from the amino acid No. 1 to the amino acid No. 99 is an amino acid sequence of human MCF precursor (corresponding to the amino acid sequence represented by the formula [II]), and an amino acid sequence from the amino acid No. 24 to the amino acid No. 99 is an amino acid sequence of human MCF (corrresponding to the amino acid sequence represented by the formula [I]). An amino acid (X) at the amino acid No. 76 means Ala or Thr.

### EXAMPLE 2

### Production of human MCF polypeptide

### (1) Construction of an expression plasmid pHMCO76

An expression plasmid for producing a polypeptide consisting of an amino acid sequence from the 24th position to the 99th position of human MCF precursor polypeptide shown in Table 1, corresponding to the amino acid sequence represented by the formula [I] (wherein X is Ala) was constructed by the following methods.

From the recombinant plasmid pHMCF7 mentioned in Example 1, a larger DNA fragment containing the nucleotide sequence encoding the entire human MCF polypeptide was isolated by digestion with restriction endonuclease PstI. This DNA fragment was then cloned into a phage vector M13mp18 (Takara Shuzo Co.) at PstI cleavage site in its polylinker sequence. By using the resulting recombinant phage DNA, a specific nucleotide sequence being 5'-TTTAAATTATG-3' was inserted between the codon corresponding to Ala at the 23rd position from the N-terminus of human MCF precursor polypeptide and the codon corresponding to Gln at the 24th position, and a specific nucleotide sequence being 5'-TGACTCGAG-3' was inserted between the translation stop codon (TGA) connected to the codon corresponding to Thr of the C-terminus of said precursor polypeptide and the 3'-untranslated nucleotide sequence, by the technique of site-directed mutagenesis according to the method of Kunkel et al. (Methods in Enzymol., 154, 367, 1987). The site-directed mutagenesis was carried out using a Muta-Gene in vitro mutanegesis kit according to the instruction manual (Bio-Rad Labs.). Namely, E. coli JM105 was infected with the recombinant phage DNA, and then it was cultivated to collect the recombinant phage. Then, E. coli CJ236 was infected with the recombinant phage obtained as above and cultivated in 2xTY medium [composition; 1.6% tryptone, 1% yeast extract, 0.5% NaCl] supplemented with uridine (1 microgram/ml) and chloramphenicol (20 micrograms/ml) at 37°C for 5 hours. The single-stranded phage DNA containing uracils was isolated from the culture medium.

Separately, two kinds of mutagenic oligodeoxyribonucleotide primers represented by the following formulae [5] and [6] were chemically synthesized.

5'-CAAGGGCTCGCTTTTAAATTATGCAGCCAGATGC-3'
formula [5]

5'-CCGAAGACTTGATGACTCGAGACACTCACTCCAC-3'
formula [6]

The 5'-terminus of each mutagenic primer was previously phosphorylated. The phosphorylated primer was annealed with the single-stranded phage DNA containing uracils prepared as above in an anneal buffer [composition: 20mM Tris-HCl buffer(pH 7.4) containing 2mM magnesium chloride and 50nM NaCl] by incubating at 70°C for 10 minutes, followed by cooling down to 30°C at a rate of 1°C per minute. Then, the primer was extended with T4 DNA polymerase in a synthesis buffer [composition: 10mM Tris-HCl buffer (pH 7.4) containing 0.4mM each deoxynucleoside triphosphate (dGTP, dATP, dCTP, dTTP), 0.75mM ATP, 3.75mM magnesium chloride and 1.5mM DTT] to synthesize a complementary strand and the ends was ligated with T4 DNA ligase by sequential incubating on ice for 5 minutes, at 25°C for 5 minutes and at 37°C for 90 minutes. The reaction was stopped by freezing at -20°C. The circular double-stranded DNA obtained as above was introduced into E. coli JM105, and they were cultivated to isolate the mutated double-stranded replicative form DNA. The nucleotide sequence of the mutated DNA was confirmed by sequencing (dideoxy method) the single-stranded DNA isolated from the culture medium.

The resulting mutated double-stranded DNA was digested with restriction endonucleases DraI and XhoI in order to isolate a DNA fragment containing the coding region for human MCF polypeptide. The isolated DNA fragment is, hereinafter, referred to as the "MCF(DraI-XhoI)-fragment".

Separately, an expression plasmid pEP205 as mentioned in Referential Example 1 was digested with restriction endonucleases DraI and XhoI, and the resulting larger DNA fragment including an amplicillin-resistance gene and a replication origin (hereinafter referred to as the "EP205 vector-DNA fragment") was isolated, and this EP205 vector-DNA fragment was ligated by T4 DNA ligase with the MCF(DraI-XhoI)-fragment previously prepared in order to construct an expression plasmid pHMC076 for producing human MCF.

The resulting expression plasmid pHMC076 was introduced into E. coli HB101 according to the method mentioned in Example 1.

E. coli HB101 transformed with the expression plasmid was cultivated on the LB agar plates (agar concentration: 1.5%) containing 25 micrograms/ml of amplicillin. After cultivation at 37°C overnight, amplicillin-resistant colonies were selected to obtain transformants. One of the amplicillin-resistant clones, a transformant, was named E. coli HB101/pHMC076 and it was used for producing the human MCF polypeptide consisting of an amino acid sequence represented by the formula [I] (wherein X is Ala).

### (2) Production of human MCF polypeptide

E. coli HB101/pHMC076 obtained as above was cultivated in LB broth overnight at 37°C. The culture was inoculated in 100-fold volumes of a nutrient medium [composition; 1.5% sodium phosphate, dibasic 12-water, 0.3% potassium phosphate, monobasic, 0.1% ammonium chloride, 2 mg/liter vitamine B1, 0.5% casamino acids, 2mM magnesium sulfate, 0.1mM calcium chloride, 1% tryptone, 0.5% yeast extract, 1% NaCl and 0.4% glycerol] and then, 3-indoleacrylic acid was added to a final concentration of 20 micrograms/ml. The cultivation was done at 35 to 37°C for 20 to 30 hours. The cells were collected by centrifugation, and suspended in 50mM Tris-HCl buffer (pH 8.0) containing 0.1% lysozyme and 30mM NaCl. The suspension was allowed to stand in an ice-water for 30 minutes. Further, freezing in a dryice/ethanol bath and thawing at 37°C were repeated to disrupt the cells. After adding 1/50 volume of 10% ethyleneimine polymer, a clarified cell-extract was obtained by centrifugation. To this cell-extract, ammonium sulfate was added to a 70% saturation, and the formed precipitate was collected by centrifugation. The precipitate was dissolved in distilled water and then it is dialyzed against 5mM phosphate buffered saline (pH 6.5). The dialysate was applied onto a column of Sephacryl S-200 (Pharmacia), and the fractions containing human MCF polypeptide were collected by judging from SDS-polyacrylamide gel electrophoretic analysis and monocyte chemotactic activity, and pooled. The pooled fraction was dialyzed against 20mM phosphate buffer (pH 6.5), and then the dialysate was applied onto a column of CM-Sepharose (Pharmacia) previously equilibrated with the same buffer. Human MCF polypeptide was eluted from the column with a gradient of NaCl molarity (0 to 0.5M). The fractions containing human MCF polypeptide were collected and pooled, and concentrated by ultrafiltration. Further, the concentrate was subjected to gel filtration on Toyopearl HW-55 column (TOSOH Co., Japan) to obtain the purified human MCF polypeptide.

### (3) Production of human MCF polypeptide

The transformant (E. coli HB101/pHMC076) obtained as above was cultivated. Then the expression plasmid pHMC076 was isolated from the transformant by a conventional manner and purified by ultracentrifugation to equilibrium in cesium chloride-ethidium bromide gradients (Maniatis, T. et al., Molecular Cloning: A Laboratory Manual. Cold Spring Harbor Laboratory, pp75-96, 1982). Human MCF polypeptide was produced by using the above expression plasmid DNA with a Prokaryotic DNA-Directed Translation Kit (Code No. N.380; Amershem Intl. plc, UK). Production of a polypeptide using the expression plasmid and the above translation kit was done according to the instruction manual (Amersham Intl. plc), if necessary by adding a RNase inhibitor (from human placenta; Amersham Intl. plc).

It was confirmed by the method mentioned previously that the human MCF polypeptide produced as above showed chemotactic activity for human monocytes.

Human MCF polypeptide was purified by the methods described in above (2).

Molecular weight of human MCF polypeptide was determined to be approximately 13±1 kDa by SDS-polyacrylamide gel electrophoresis.

### EXAMPLE 3

### Production of polypeptide having human MCF activity

Some expression plasmids for producing a polypeptide with human MCF activity consisting of an amino acid sequence in which an amino acid at the 27th position or the 30th position from the N-terminus of human MCF precursor polypeptide shown in Table 1 (wherein X is Ala) is the N-terminus, that is a polypeptide truncated its N-terminal region from human MCF polypeptide mentioned in Example 2, were constructed.

Namely, from the expression plasmid pHMC076 mentioned in Example 2, a DNA fragment containing the MCF(DraI-XhoI)-fragment mentioned in Example 2 was isolated by digestion with restriction endonucleases SpeI and SalI. This DNA fragment was then cloned into a phage vector M13mp19 (Takara Shuzo Co.) at a region between the restriction endonuclease cleavage site of SalI and that of XbaI in its polylinker sequence. By using the resulting recombinant phage DNA as a template and some mutagenic primers as shown below, a nucleotide sequence coding for the N-terminal amino acids of human MCF polypeptide was deleted from the MCF(DraI-XhoI)-fragment by the technique of site-directed mutagenesis mentioned in Example 2. The resulting deleted DNA fragment was ligated with the EP205 vector-DNA fragment mentioned in Example 2 to construct some expression plasmids.

A nucleotide sequence of the mutagenic primer used for site-directed mutagenesis was as follows.

In the case of constructing an expression plasmid for producing a polypeptide consisting of an amino acid sequence from the 27th position to the 99th position of human MCF precursor polypeptide shown in Table 1 (wherein X is Ala) (hereinafter abbreviated as the "N3-MCF polypeptide"):

5'-GGTTTAAATTATGGCAATCAATGCCC-3'

In the case of constructing an expression plasmid for producing a polypeptide consisting of an amino acid sequence from the 30th position to the 99th position of human MCF precursor polypeptide (X in Table 1 is Ala) (hereinafter abbreviated as the "N6-MCF polypeptide"):

5'-GGTTTAAATTATGGCCCCAGTCACCTGC-3'

According to the method mentioned in Example 2, each mutated double-stranded replicative form DNA was prepared and isolated. Then each resulting mutated double-stranded DNA was digested with restriction endonucleases DraI and XhoI in order to isolate each DNA fragment containing the coding region for the desired polypeptide. An expression plasmid for producing the desired polypeptide was constructed by ligating each of these DNA fragments with the EP205 vector-DNA fragment derived from an expression vector pEP205.

An expression plasmid for producing the N3-MCF polypeptide was designated pHMC073, and an expression plasmid for producing the N6-MCF polypeptide was designated pHMC070.

### (2) Production of a polypeptide having human MCF activity

The expression plasmid constructed as above was introduced into E. coli HB101 according to the method mentioned in Example 2. Furthermore, by cultivating the resulting transformant, a polypeptide with human MCF activity was produced according to the method mentioned in Example 2.

By using the above expression plasmid, a polypeptide was synthesized in vitro with a Prokaryotic DNA-Directed Translation Kit (Amersham Intl. plc, UK). The expression plasmid was isolated by a conventional manner and purified by ultracentrifugation to equilibrium in cesium chloride-ethidium bromide gradients (Maniatis, T. et al., Molecular Cloning: A Laboratory Manual. Cold Spring Harbor Laboratory, pp75-96, 1982). Production of a polypeptide using the expression plasmid and the above translation kit was done according to the instruction manual (Amersham Intl. plc), by using a tritium-labelled leucine and adding a RNase inhibitor (from human placenta; Amersham Intl. plc) into the transcription/translation reaction mixture. The product was identified by an autoradiography of SDS-polyacrylamide gel electrophoretic pattern (Laemmli, U.K., Nature, 227, 680, 1970), or on the basis of biological activity.

### (3) Production of a polypeptide having human MCF activity

The transformant obtained as above was cultivated and the expression plasmid was isolated according to the method mentioned in Example 2. Furthermore, by using each of the above expression plasmid, N3-MCF polypeptide and N6-polypeptide were produced according to the method mentioned in Example 2. It was confirmed that these polypeptide had chemotactic activity for human monocytes.

Both molecular weights of N3-MCF polypeptide and N6-MCF polypeptide were determined to be approximately 12±1 kDa by SDS-polyacrylamide gel electrophoresis. Since the mobility of the former polypeptide was a little slower than that of the latter polypeptide, however, the former polypeptide was somewhat higher molecular weight.

### REFERENTIAL EXAMPLE 1

### Construction of an expression vector pEP205

Plasmid pBR322 was digested with restriction endonucleases AvaI and PvuII, and the resulting larger DNA fragment (about 3.7 kbp in size) was isolated. After filling-in its cohesive ends to blunt-ends with E. coli DNA polymerase I (Klenow fragment) in the presence of dGTP, dATP, dCTP and dTTP, both ends were ligated by T4 DNA ligase to construct a new plasmid vector (designated pBRS6), which was deleted a copy number regulatory gene region located near the replication origin of the plasmid pBR322.

The plasmid vector pBRS6 was digested with restriction endonucleases EcoRI and PstI, and a smaller DNA fragment containing an upstream region of the ampicillin-resistance gene (about 0.75 kbp in size) was isolated. The resulting DNA fragment is referred to as the "Amp(PstI-EcoRI)-fragment".

This Amp(PstI-EcoRI)-fragment was cloned in a phage vector M13mp18 as mentioned in Example 2. By using the resulting recombinant phage DNA, one base (A) in the nucleotide sequence of the Amp(PstI-ECORI)-fragment was changed to another base (G) by the site-directed mutagenesis according to the method as mentioned in Example 2, in order to eliminate the specific nucleotide sequence (TTTAAA) recognizable with the restriction endonuclease DraI.

Namely, the single-stranded phage DNA containing uracils was isolated from the culture medium of E. coli CJ236 infected with the above recombinant phage DNA.

As a mutagenic primer, the oligodeoxyribonucleotide represented by the following formula [7] was chemically synthesized.

5'-CAGAACTTTGAAAGTGCTC-3'    formula [7]

The phosphorylated primer was annealed with the uracil-containing DNA template. According to the method described in Example 2, the desired mutated double-stranded DNA was isolated.

The resulting mutated double-stranded DNA was digested with restriction endonucleases PstI and EcoRI in order to isolate a DNA fragment corresponding to the Amp(PstI-EcoRI)-fragment as mentioned above, but not containing the restriction endonuclease DraI cleavage recognition sequence [hereinafter referred to as the "mutated Amp(PstI-EcoRI)-fragment"]. The mutated Amp(PstI-EcoRI)-fragment was ligated with the larger DNA fragment isolated from the vector pBRS6 by digestion with restriction endonucleases EcoRI and PstI, in order to construct a new vector which was eliminated the DraI cleavage recognition sequence in the ampicillin resistance gene of the plasmid vector pBRS6. This new vector is designated pBRS601.

Further, this new vector pBRS601 was digested with restriction endonuclease DraI, and the resulting larger DNA fragment was isolated. The larger DNA fragment was ligated with SmaI linker (Takara Shuzo Co.) by T4 DNA ligase to construct a new plasmid vector. This resulting new plasmid vector is a derivative of plasmid pBRS6 and is not containing any recognition sequences for the restriction endonuclease DraI. This new plasmid vector is designated pBRS602. The nucleotide sequence of the SmaI linker is shown below.

5'-CCCGGG-3'

Furthermore, this new vector pBRS602 was digested with restriction endonucleases AatII and SalI, and the resulting larger DNA fragment was isolated [hereinafter referred to as the "pBRS602(AatII-SalI)-fragment"].

Separately, an expression plasmid pHIPH383a for producing human interleukin-1α as mentioned in Referential Example 2, was digested with restriction endonucleases AatII and SalI, and the resulting DNA fragment containing E. coli tryptophan promoter sequence and the coding region for human interleukin-1 was isolated. This resulting DNA fragment is referred to as the "trp promoter/IL1α-DNA fragment".

This trp promoter/IL1α-DNA fragment was ligated with the pBRS602(AatII-SalI)-fragment by T4 DNA ligase to construct a new expression plasmid. This new expression plasmid is designated pEP205.

### REFERENTIAL EXAMPLE 2

### Construction of an expression plasmid pHIPH383a

The cloned cDNA encoding human interleukin-1 precursor polypeptide was isolated according to the method described in European Patent Publication No. 0188920. From the recombinant plasmid pHL4 containing human interleukin-1 cDNA (Furutani,Y., et al., Nucleic Acids Res., 13, 5869, 1985), the cDNA insert was isolated by digestion with restriction endonuclease PstI, and further digested with restriction endonucleases EcoRI and BstNI, to isolate a DNA fragment (about 411 bp in size) containing a middle portion of the coding region for the mature human interleukin-1α. The isolated DNA fragment is corresponding to the nucleotide sequence from the base No. 398 to the base No. 808 in Table 5 shown in European Patent Publication No. 0188920.

This DNA fragment was sequentially ligated by T4 DNA ligase with chemically synthesized oligodeoxyribonucleotide adaptors represented by the following formulae [8] and [9]. The resulting DNA fragment is referred to as the "SD-IL1-frgment".

The synthetic oligodeoxyribonucleotide adaptor [8] was prepared by sequential ligation of the following five kinds of DNA fragments represented by formuale [a] to [e]. 
5'-AACTAGTACGCAAGTTCAC
3'-TTGATCATGCGTTCAAGTGCATT [a]
 
5'-GTAAAAGGAGGTTTAAA
3'-TTCCTCCAAATTTAATAC [b]
 
5'-TTATGTCATCACCTTTTAG
3'-AGTAGTGGAAAATCGAAGG [C]
 
5'-CTTCCTGAGCAATGTGAAATACAACTTTA
3'-ACTCGTTACACTTTATGTTGAAATACTC [d]
 and

5'-TGAGGATCATCAAATACG
    3'-CTAGTAGTTTATGCTTAA    [e]

A nucleotide sequence of the formula [9] was as follows:

5'-AGGCGTGATGACTCGA
 3'-CCGCACTACTGAGCTCTAG     formula [9]

Separately, an expression vector pEP302 (Furutani, Y., et al., Nucleic Acids Res., 13, 5869, 1985) was digested with restriction endonucleases HpaI and BamHI, and the resulting larger DNA fragment containing E. coli tryptophan promoter sequence and an ampicillin resistance gene, was isolated (hereinafter referred to as the "EP302 vector-DNA fragment").

The EP302 vector-DNA fragment was ligated by T4 DNA ligase with the SD-IL1-fragment prepared as above to construct an expression plasmid pHIPH383a for producing the mature human interleukin-1α polypeptide.

## Claims (Claims for the following Contracting State(s): AT, BE, CH, LI, DE, DK, FR, GB, IT, LU, SE, NL)

1. An isolated DNA sequence coding for the expression of a polypeptide with human monocyte chemotactic factor activity comprising amino acid sequence [I] or any portion of said amino acid sequence capable of exhibiting human monocyte chemotactic factor activity 
Gln Pro Asp Ala Ile Asn Ala Pro Val Thr
Cys Cys Tyr Asn Phe Thr Asn Arg Lys Ile
Ser Val Gln Arg Leu Ala Ser Tyr Arg Arg
Ile Thr Ser Ser Lys Cys Pro Lys Glu Ala
Val Ile Phe Lys Thr Ile Val Ala Lys Glu
Ile Cys X Asp Pro Lys Gln Lys Trp Val
Gln Asp Ser Met Asp His Leu Asp Lys Gln
Thr Gln Thr Pro Lys Thr
[I]
 (wherein X means Ala or Thr).

2. An isolated DNA sequence, as claimed in claim 1, having the nucleotide sequence represented by the formula A 
CAGCCAGATGCAATCAATGCCCCAGTCACC
TGCTGYTATAACTTCACCAATAGGAAGATC
TCAGTGCAGAGGCTCGCGAGCTATAGAAGA
ATCACCAGCAGCAAGTGTCCCAAAGAAGCT
GTGATCTTCAAGACCATTGTGGCCAAGGAG
ATCTGTRCTGACCCCAAGCAGAAGTGGGTT
CAGGATTCCATGGACCACCTGGACAAGCAA
ACCCAAACTCCGAAGACT formula [A]
 (wherein Y means C or T, and R means G or A).

3. An isolated DNA sequence deleted 23 to 29 codons from the 5'-terminus of a DNA coding for the expression of a human monocyte chemotactic factor precursor comprising amino acid sequence [II] or its allelic mutant DNA 
Met Lys Val Ser Ala Ala Leu Leu Cys Leu
Leu Leu Ile Ala Ala Thr Phe Ile Pro Gln
Gly Leu Ala Gln Pro Asp Ala Ile Asn Ala
Pro Val Thr Cys Cys Tyr Asn Phe Thr Asn
Arg Lys Ile Ser Val Gln Arg Leu Ala Ser
Tyr Arg Arg Ile Thr Ser Ser Lys Cys Pro
Lys Glu Ala Val Ile Phe Lys Thr Ile Val
Ala Lys Glu Ile Cys X Asp Pro Lys Gln
Lys Trp Val Gln Asp Ser Met Asp His Leu
Asp Lys Gln Thr Gln Thr Pro Lys Thr

[II]
 (wherein X means Ala or Thr).

4. An isolated DNA sequence as claimed in claim 3 wherein said DNA coding for the expression of a human monocyte chemotactic precursor has a nucleotide sequence represented by one of the following formulae 
ATGAAAGTCTCTGCCGCCCTTCTGTGCCTG 30
MetLysValSerAlaAlaLeuLeuCysLeu (10)
CTGCTCATAGCAGCCACCTTCATTCCCCAA 60
LeuLeuIleAlaAlaThrPheIleProGln (20)
GGGCTCGCTCAGCCAGATGCAATCAATGCC 90
GlyLeuAlaGlnProAspAlaIleAsnAla (30)
CCAGTCACCTGCTGTTATAACTTCACCAAT 120
ProValThrCysCysTyrAsnPheThrAsn (40)
AGGAAGATCTCAGTGCAGAGGCTCGCGAGC 150
ArgLysIleSerValGlnArgLeuAlaSer (50)
TATAGAAGAATCACCAGCAGCAAGTGTCCC 180
TyrArgArgIleThrSerSerLysCysPro (60)
AAAGAAGCTGTGATCTTCAAGACCATTGTG 210
LysGluAlaValIlePheLysThrIleVal (70)
GCCAAGGAGATCTGTRCTGACCCCAAGCAG 240
AlaLysGluIleCys X AspProLysGln (80)
AAGTGGGTTCAGGATTCCATGGACCACCTG 270
LysTrpValGlnAspSerMetAspHisLeu (90)
GACAAGCAAACCCAAACTCCGAAGACTTGA 300
AspLysGlnThrGlnThrProLysThr*** (99)
ACACTCACTCCACAACCCAAGAATCTGCAG 330
 (wherein Y is C or T and R is G or A).

5. A vector containing a DNA sequence as claimed in any one of the preceding claims.

6. A microbial host transformed by a vector as claimed in claim 5.

7. A process for producing a recombinant polypeptide with human monocyte chemotactic factor activity comprising culturing a transformed microbial host as claimed in claim 6 under conditions suitable for expression of a polypeptide with human monocyte chemotactic factor activity and recovering said polypeptide.

8. A purified polypeptide with human monocyte chemotactic factor activity obtainable by a process as claimed in claim 7.

9. An essentially pure polypeptide with human monocyte chemotactic factor activity consisting of amino acid sequence [I] as shown in claim 1 or any portion of said amino acid sequence capable of exhibiting human monocyte chemotactic factor activity.

10. An essentially pure polypeptide with human monocyte chemotactic factor activity as claimed in claim 9, in which the N-terminal 1 to 6 amino acids are deleted.

## Claims (Claims for the following Contracting State(s): ES)

1. A process for isolating a DNA sequence coding for the expression of a polypeptide with human monocyte chemotactic factor activity comprising amino acid sequence [I] or any portion of said amino acid sequence capable of exhibiting human monocyte chemotactic factor activitv 
Gln Pro Asp Ala Ile Asn Ala Pro Val Thr
Cys Cys Tyr Asn Phe Thr Asn Arg Lys Ile
Ser Val Gln Arg Leu Ala Ser Tyr Arg Arg
Ile Thr Ser Ser Lys Cys Pro Lys Glu Ala
Val Ile Phe Lys Thr Ile Val Ala Lys Glu
Ile Cys X Asp Pro Lys Gln Lys Trp Val
Gln Asp Ser Met Asp His Leu Asp Lys Gln
Thr Gln Thr Pro Lys Thr
[I]
 (wherein X means Ala or Thr),
said process involving the use of a recombinant DNA technique.

2. A process as claimed in claim 1, wherein the isolated DNA sequence has the nucleotide sequence represented by the formula A 
CAGCCAGATGCAATCAATGCCCCAGTCACC
TGCTGYTATAACTTCACCAATAGGAAGATC
TCAGTGCAGAGGCTCGCGAGCTATAGAAGA
ATCACCAGCAGCAAGTGTCCCAAAGAAGCT
GTGATCTTCAAGACCATTGTGGCCAAGGAG
ATCTGTRCTGACCCCAAGCAGAAGTGGGTT
CAGGATTCCATGGACCACCTGGACAAGCAA
ACCCAAACTCCGAAGACT formula (A)
 (wherein Y means C or T, and R means G or A).

3. A process for isolating a DNA sequence deleted 23 to 29 codons from the 5'-terminus of a DNA coding for the expression of a human monocyte chemotactic factor precursor comprising amino acid sequence [II] or its allelic mutant DNA 
Met Lys Val Ser Ala Ala Leu Leu Cys Leu
Leu Leu Ile Ala Ala Thr Phe Ile Pro Gln
Gly Leu Ala Gln Pro Asp Ala Ile Asn Ala
Pro Val Thr Cys Cys Tyr Asn Phe Thr Asn
Arg Lys Ile Ser Val Gln Arg Leu Ala Ser
Tyr Arg Arg Ile Thr Ser Ser Lys Cys Pro
Lys Glu Ala Val Ile Phe Lys Thr Ile Val
Ala Lys Glu Ile Cys X Asp Pro Lys Gln
Lys Trp Val Gln Asp Ser Met Asp His Leu
Asp Lys Gln Thr Gln Thr Pro Lys Thr
[II]
 (wherein X means Ala or Thr), said process involving the use of a recombinant DNA technique.

4. A process as claimed in claim 3, wherein said DNA coding for the expression of a human monocyte chemotactic precursor has a nucleotide sequence represented by one of the following formulae 
ATGAAAGTCTCTGCCGCCCTTCTGTGCCTG 30
MetLysValSerAlaAlaLeuLeuCysLeu (10)
CTGCTCATAGCAGCCACCTTCATTCCCCAA 60
LeuLeuIleAlaAlaThrPheIleProGln (20)
GGGCTCGCTCAGCCAGATGCAATCAATGCC 90
GlyLeuAlaGlnProAspAlaIleAsnAla (30)
CCAGTCACCTGCTGYTATAACTTCACCAAT 120
ProValThrCysCysTyrAsnPheThrAsn (40)
AGGAAGATCTCAGTGCAGAGGCTCGCGAGC 150
ArgLysIleSerValGlnArgLeuAlaSer (50)
TATAGAAGAATCACCAGCAGCAAGTGTCCC 180
TyrArgArgIleThrSerSerLysCysPro (60)
AAAGAAGCTGTGATCTTCAAGACCATTGTG 210
LysGluAlaValIlePheLysThrIleVal (70)
GCCAAGGAGATCTGTRCTGACCCCAAGCAG 240
AlaLysGluIleCys X AspProLysGln (80)
AAGTGGGTTCAGGATTCCATGGACCACCTG 270
LysTrpValGlnAspSerMetAspHisLeu (90)
GACAAGCAAACCCAAACTCCGAAGACTTGA 300
AspLysGlnThrGlnThrProLysThr*** (99)
ACACTCACTCCACAACCCAAGAATCTGCAG 330
 (wherein Y is C or T and R is G or A).

5. A process for constructing a vector containing a DNA sequence as defined in any one of the preceding claims, said process involving the use of a recombinant DNA technique.

6. A process for producing a microbial host transformed by a vector as defined in claim 5, comprising the step of introducing a vector into a microbial host which it is desired to transform.

7. A process for producing a recombinant polypeptide with human monocyte chemotactic factor activity comprising producing a transformed microbial host as claimed in claim 6, culturing said microbial host under conditions suitable for expression of a polypeptide with human monocyte chemotactic factor activity and recovering said polypeptide.

8. A process for producing a purified polypeptide with human monocyte chemotactic factor activity, said process involving the use of a protein purification technique.

9. A process for producing an essentially pure polypeptide with human monocyte chemotactic factor activity consisting of amino acid sequence [I] as shown in claim 1 or any portion of said amino acid sequence capable of exhibiting human monocyte chemotactic factor activity, said process involving the use of a protein purification technique.

10. A process for producing an essentially pure polypeptide with human monocyte chemotactic factor activity as claimed in claim 9, in which the N-terminal 1 to 6 amino acids are deleted.

## Patentansprüche (Patentansprüche für folgende(n) Vertragsstaat(en): AT, BE, CH, LI, DE, DK, FR, GB, IT, LU, SE, NL)

1. Isolierte DNA-Sequenz, welche für die Expression eines Polypeptids mit chemotaktischer Faktorakivität menschlicher 5 Monozyten kodiert, bestehend aus der Aminosäuresequenz (I) oder irgendeines Teils dieser Aminosäuresequenz, die chemotaktische Faktoraktivität menschlicher Monozyten zeigen kann 
Gln Pro Asp Ala Ile Asn Ala Pro Val Thr
Cys Cys Tyr Asn Phe Thr Asn Arg Lys Ile
Ser Val Gln Arg Leu Ala Ser Tyr Arg Arg
Ile Thr Ser Ser Lys Cys Pro Lys Glu Ala
Val Ile Phe Lys Thr Ile Val Ala Lys Glu
Ile Cys X Asp Pro Lys Gln Lys Trp Val
Gln Asp Ser Met Asp His Leu Asp Lys Gln
Thr Gln Thr Pro Lys Thr
[I]
 (wobei X die Bedeutung Ala oder Thr hat).

2. Isolierte DNA-Sequenz nach Anspruch 1, mit der durch Formel A repräsentierten Nucleotidsequenz 
CAGCCAGATGCAATCAATGCCCCAGTCACC
TGCTGYTATAACTTCACCAATAGGAAGATC
TCAGTGCAGAGGCTCGCGAGCTATAGAAGA
ATCACCAGCAGCAAGTGTCCCAAAGAAGCT
GTGATCTTCAAGACCATTGTGGCCAAGGAG
ATCTGTRCTGACCCCAAGCAGAAGTGGGTT
CAGGATTCCATGGACCACCTGGACAAGCAA
ACCCAAACTCCGAAGACT
Formel A
 (wobei Y die Bedeutung C oder T hat, und R die Bedeutung G oder A hat).

3. Isolierte DNA-Sequenz, die 23 bis 29 Codons vom 5'-Ende einer DNA deletiert ist, die für die Expression eines chemotaktischen Faktorvorläufers menschlicher Monozyten kodiert, bestehend aus der Aminosäuresequenz (II) oder ihrer allelen Mutanten-DNA 
Met Lys Val Ser Ala Ala Leu Leu Cys Leu
Leu Leu Ile Ala Ala Thr Phe Ile Pro Gln
Gly Leu Ala Gln Pro Asp Ala Ile Asn Ala
Pro Val Thr Cys Cys Tyr Asn Phe Thr Asn
Arg Lys Ile Ser Val Gln Arg Leu Ala Ser
Tyr Arg Arg Ile Thr Ser Ser Lys Cys Pro
Lys Glu Ala Val Ile Phe Lys Thr Ile Val
Ala Lys Glu Ile Cys X Asp Pro Lys Gln
Lys Trp Val Gln Asp Ser Met Asp His Leu
Asp Lys Gln Thr Gln Thr Pro Lys Thr

[II]
 (wobei X die Bedeutung Ala oder Thr hat).

4. Isolierte DNA-Sequenz nach Anspruch 3, wobei diese DNA für die Expression eines chemotaktischen Aktivitätsvorläufers menschlicher Monozyten kodiert, der eine Nucleotidsequenz aufweist, die durch eine der folgenden Formeln repräsentiert ist 
ATGAAAGTCTCTGCCGCCCTTCTGTGCCTG 30
MetLysValSerAlaAlaLeuLeuCysLeu (10)
CTGCTCATAGCAGCCACCTTCATTCCCCAA 60
LeuLeuIleAlaAlaThrPheIleProGln (20)
GGGCTCGCTCAGCCAGATGCAATCAATGCC 90
GlyLeuAlaGlnProAspAlaIleAsnAla (30)
CCAGTCACCTGCTGYTATAACTTCACCAAT 120
ProValThrCysCysTyrAsnPheThrAsn (40)
AGGAAGATCTCAGTGCAGAGGCTCGCGAGC 150
ArgLysIleSerValGlnArgLeuAlaSer (50)
TATAGAAGAATCACCAGCAGCAAGTGTCCC 180
TyrArgArgIleThrSerSerLysCysPro (60)
AAAGAAGCTGTGATCTTCAAGACCATTGTG 210
LysGluAlaValIlePheLysThrIleVal (70)
GCCAAGGAGATCTGTRCTGACCCCAAGCAG 240
AlaLysGluIleCys X AspProLysGln (80)
AAGTGGGTTCAGGATTCCATGGACCACCTG 270
LysTrpValGlnAspSerMetAspHisLeu (90)
GACAAGCAAACCCAAACTCCGAAGACTTGA 300
AspLysGlnThrGlnThrProLysThr*** (99)
ACACTCACTCCACAACCCAAGAATCTGCAG 330
 (wobei Y die Bedeutung C oder T hat, und R die Bedeutung G oder A hat).

5. Vektor, der eine DNA-Sequenz nach einem der vorhergehenden Ansprüche enthält.

6. Mikrobenwirt, der durch einen Vektor nach Anspruch 5 transformiert ist.

7. Verfahren zur Herstellung eines rekombinanten Polypeptids mit chemotaktischer Faktoraktivität menschlicher Monozyten, welches das Kultivieren eines transformierten Mikrobenwirts nach Anspruch 6 unter Bedingungen, die für die Expression eines Polypeptids mit chemotaktischer Faktoraktivität menschlicher Monozyten geeignet sind, und das Herausgewinnen dieses Polypeptids umfaßt.

8. In reiner Form gewonnenes Polypeptid mit chemotaktischer Faktoraktivität menschlicher Monozyten, wie es durch ein Verfahren nach Anspruch 7 gewinnbar ist.

9. Im wesentlichen reines Polypeptid mit chemotaktischer Faktoraktivität menschlicher Monozyten, bestehend aus der Aminosäuresequenz (I), wie in Anspruch 1 dargestellt, oder irgend einem Teil dieser Aminosäuresequenz, die chemotaktische Faktoraktivität menschlicher Monozyten zeigen kann.

10. Im wesentlichen reines Polypeptid mit chemotaktischer Faktoraktivität menschlicher Monozyten nach Anspruch 9, wobei die N-terminalen 1 bis 6 Aminosäuren deletiert sind.

## Patentansprüche (Patentansprüche für folgende(n) Vertragsstaat(en): ES)

1. Verfahren zum Isolieren einer DNA-Sequenz, welche für die Expression eines Polypeptids mit chemotaktischer Faktoraktivität menschlicher Monozyten kodiert, bestehend aus der Aminosäuresequenz (I) oder irgendeines Teils dieser Aminosäuresequenz, die chemotaktische Faktoraktivität menschlicher Monozyten zeigen kann 
Gln Pro Asp Ala Ile Asn Ala Pro Val Thr
Cys Cys Tyr Asn Phe Thr Asn Arg Lys Ile
Ser Val Gln Arg Leu Ala Ser Tyr Arg Arg
Ile Thr Ser Ser Lys Cys Pro Lys Glu Ala
Val Ile Phe Lys Thr Ile Val Ala Lys Glu
Ile Cys X Asp Pro Lys Gln Lys Trp Val
Gln Asp Ser Met Asp His Leu Asp Lys Gln
Thr Gln Thr Pro Lys Thr
[I]
 (wobei X die Bedeutung Ala oder Thr hat), wobei das Verfahren die Anwendung einer rekombinanten DNA-Technik beinhaltet.

2. Verfahren nach Anspruch 1, bei dem die isolierte DNA-Sequenz die Nucleotidsequenz hat, die repräsentiert wird durch Formel A: 
CAGCCAGATGCAATCAATGCCCCAGTCACC
TGCTGYTATAACTTCACCAATAGGAAGATC
TCAGTGCAGAGGCTCGCGAGCTATAGAAGA
ATCACCAGCAGCAAGTGTCCCAAAGAAGCT
GTGATCTTCAAGACCATTGTGGCCAAGGAG
ATCTGTRCTGACCCCAAGCAGAAGTGGGTT
CAGGATTCCATGGACCACCTGGACAAGCAA
ACCCAAACTCCGAAGACT Formel A
 (wobei Y die Bedeutung C oder T und R die Bedeutung G oder A hat).

3. Verfahren zum Isolieren einer DNA-Sequenz, die 23 bis 29 Codons vom 5'-Ende einer DNA deletiert ist, die für die Expression eines chemotaktischen Faktorvorläufers menschlicher Monzyten kodiert, bestehend aus der Aminosäuresequenz (II) oder ihrer allelen Mutanten-DNA 
Met Lys Val Ser Ala Ala Leu Leu Cys Leu
Leu Leu Ile Ala Ala Thr Phe Ile Pro Gln
Gly Leu Ala Gln Pro Asp Ala Ile Asn Ala
Pro Val Thr Cys Cys Tyr Asn Phe Thr Asn
Arg Lys Ile Ser Val Gln Arg Leu Ala Ser
Tyr Arg Arg Ile Thr Ser Ser Lys Cys Pro
Lys Glu Ala Val Ile Phe Lys Thr Ile Val
Ala Lys Glu Ile Cys X Asp Pro Lys Gln
Lys Trp Val Gln Asp Ser Met Asp His Leu
Asp Lys Gln Thr Gln Thr Pro Lys Thr

[II]
 (wobei X die Bedeutung Ala oder Thr hat), wobei das Verfahren die Anwendung einer rekombinanten DNA-Technik beinhaltet.

4. Verfahren nach Anspruch 3, bei dem die genannte DNA, die für die Expression eines chemotaktischen Aktivitätsvorläufers menschlicher Monozyten kodiert, eine Nucleotidsequenz aufweist, die durch eine der folgenden Formeln repräsentiert wird 
ATGAAAGTCTCTGCCGCCCTTCTGTGCCTG 30
MetLysValSerAlaAlaLeuLeuCysLeu (10)
CTGCTCATAGCAGCCACCTTCATTCCCCAA 60
LeuLeuIleAlaAlaThrPheIleProGln (20)
GGGCTCGCTCAGCCAGATGCAATCAATGCC 90
GlyLeuAlaGlnProAspAlaIleAsnAla (30)
CCAGTCACCTGCTGYTATAACTTCACCAAT 120
ProValThrCysCysTyrAsnPheThrAsn (40)
AGGAAGATCTCAGTGCAGAGGCTCGCGAGC 150
ArgLysIleSerValGlnArgLeuAlaSer (50)
TATAGAAGAATCACCAGCAGCAAGTGTCCC 180
TyrArgArgIleThrSerSerLysCysPro (60)
AAAGAAGCTGTGATCTTCAAGACCATTGTG 210
LysGluAlaValIlePheLysThrIleVal (70)
GCCAAGGAGATCTGTRCTGACCCCAAGCAG 240
AlaLysGluIleCys X AspProLysGln (80)
AAGTGGGTTCAGGATTCCATGGACCACCTG 270
LysTrpValGlnAspSerMetAspHisLeu (90)
GACAAGCAAACCCAAACTCCGAAGACTTGA 300
AspLysGlnThrGlnThrProLysThr*** (99)
ACACTCACTCCACAACCCAAGAATCTGCAG 330
 (wobei Y die Beduetung C oder T und R die Bedeutung G oder A hat).

5. Verfahren zur Konstruktion eines Vektors, der eine DNA-Sequenz nach einem der vorhergehenden Ansprüche enthält, wobei das genannte Verfahren die Anwendung einer rekombinanten DNA-Technik beinhaltet.

6. Verfahren zur Herstellung eines Mikrobenwirts, der durch einen Vektor nach Anspruch 5 transformiert ist, umfassend den Schritt des Einführens eines Vektors in einen Mikrobenwirt, der transformiert werden soll.

7. Verfahren zur Herstellung eines rekombinanten Polypeptids mit chemotaktischer Faktoraktivität menschlicher Monozyten, umfassend die Herstellung eines transformierten Mikrobenwirts nach Anspruch 6, das Kultivieren des genannten Mikrobenwirts unter Bedingungen, die für die Expression eines Polypeptids mit chemotaktischer Faktoraktivität menschlicher Monozyten geeignet sind, und das Herausgewinnen dieses Polypeptids.

8. Verfahren zur Herstellung eines purifizierten Polypeptids mit chemotaktischer Faktoraktivität menschlicher Monozyten, wobei das genannte Verfahren die Anwendung einer Proteinpurifizierungstechnik beinhaltet.

9. Verfahren zur Herstellung eines im wesentlichen reinen Polypeptids mit chemotaktischer Faktoraktivität menschlicher Monozyten, bestehend aus der Aminosäuresequenz (I), wie in Anspruch 1 dargestellt, oder irgendeinem Teil dieser Aminosäuresequenz, die chemotaktische Faktoraktivität menschlicher Monozyten zeigen kann, wobei das genannte Verfahren die Anwendung einer Proteinpurifizierungstechnik beinhaltet.

10. Verfahren zur Herstellung eines im wesentlichen reinen Polypeptids mit chemotaktischer Faktoraktivität menschlicher Monozyten nach Anspruch 9, bei dem die N-terminalen 1 bis 6 Aminosäuren deletiert sind.

## Revendications (Revendications pour l'(les) Etat(s) contractant(s) suivant(s): AT, BE, CH, LI, DE, DK, FR, GB, lT, LU, SE, NL)

1. Séquence d'ADN isolée codant pour l'expression d'un polypeptide ayant une activité de facteur chimiotactique humain pour les monocytes comprenant la séquence en acides aminés [I] ou toute partie de ladite séquence en acides aminés capable de présenter une activité de facteur chimiotactique humain pour les monocytes 
Gln Pro Asp Ala Ile Asn Ala Pro Val Thr
Cys Cys Tyr Asn Phe Thr Asn Arg Lys Ile
Ser Val Gln Arg Leu Ala Ser Tyr Arg Arg
Ile Thr Ser Ser Lys Cys Pro Lys Glu Ala
Val Ile Phe Lys Thr Ile Val Ala Lys Glu
Ile Cys X Asp Pro Lys Gln Lys Trp Val
Gln Asp Ser Met Asp His Leu Asp Lys Gln
Thr Gln Thr Pro Lys Thr [I]
 (dans laquelle X signifie Ala ou Thr).

2. Séquence d'ADN isolée, telle que revendiquée dans la revendication 1, ayant la séquence nucléotidique représentée par la formule [A] 
CAGCCAGATGCAATCAATGCCCCAGTCACC
TGCTGYTATAACTTCACCAATAGGAAGATC
TCAGTGCAGAGGCTCGCGAGCTATAGAAGA
ATCACCAGCAGCAAGTGTCCCAAAGAAGCT
GTGATCTTCAAGACCATTGTGGCCAAGGAG
ATCTGTRCTGACCCCAAGCAGAAGTGGGTT
CAGGATTCCATGGACCACCTGGACAAGCAA
ACCCAAACTCCGAAGACT formule [A]
 (dans laquelle Y signifie C ou T, et R signifie G ou A).

3. Séquence d'ADN isolée privée des 23 à 29 codons de l'extrémité 5'-terminale d'un ADN codant pour l'expression d'un précurseur de facteur chimiotactique humain pour les monocytes comprenant la séquence en acides aminés [II] ou son ADN mutant allélique 
Met Lys Val Ser Ala Ala Leu Leu Cys Leu
Leu Leu Ile Ala Ala Thr Phe Ile Pro Gln
Gly Leu Ala Gln Pro Asp Ala Ile Asn Ala
Pro Val Thr Cys Cys Tyr Asn Phe Thr Asn
Arg Lys Ile Ser Val Gln Arg Leu Ala Ser
Tyr Arg Arg Ile Thr Ser Ser Lys Cys Pro
Lys Glu Ala Val Ile Phe Lys Thr Ile Val
Ala Lys Glu Ile Cys X Asp Pro Lys Gln
Lys Trp Val Gln Asp Ser Met Asp His Leu
Asp Lys Gln Thr Gln Thr Pro Lys Thr [II]
 (dans laquelle X représente Ala ou Thr).

4. Séquence d'ADN isolée telle que revendiquée dans la revendication 3, Sans laquelle ledit ADN codant pour l'expression d'un précurseur de facteur chimiotactique humain pour les monocytes possède une séquence nucléotidique représentée par l'une des formules suivantes 
ATGAAAGTCTCTGCCGCCCTTCTGTGCCTG 30
MetLysValSerAlaAlaLeuLeuCysLeu (10)
CTGCTCATAGCAGCCACCTTCATTCCCCAA 60
LeuLeuIleAlaAlaThrPheIleProGln (20)
GGGCTCGCTCAGCCAGATGCAATCAATGCC 90
GlyLeuAlaGlnProAspAlaIleAsnAla (30)
CCAGTCACCTGCTGYTATAACTTCACCAAT 120
ProValThrCysCysTyrAsnPheThrAsn (40)
AGGAAGATCTCAGTGCAGAGGCTCGCGAGC 150
ArgLysIleSerValGlnArgLeuAlaSer (50)
TATAGAAGAATCACCAGCAGCAAGTGTCCC 180
TyrArgArgIleThrSerSerLysCysPro (60)
AAAGAAGCTGTGATCTTCAAGACCATTGTG 210
LysGluAlaValIlePheLysThrIleVal (70)
GCCAAGGAGATCTGTRCTGACCCCAAGCAG 240
AlaLysGluIleCys X AspProLysGln (80)
AAGTGGGTTCAGGATTCCATGGACCACCTG 270
LysTrpValGlnAspSerMetAspHisLeu (90)
GACAAGCAAACCCAAACTCCGAAGACTTGA 300
AspLysGlnThrGlnThrProLysThr*** (99)
 ACACTCACTCCACAACCCAAGAATCTGCAG 330 (dans laquelle Y est C ou T, et R est G ou A).

5. Vecteur contenant une séquence d'ADN telle que revendiquée dans l'une quelconque des revendications précédentes.

6. Hôte microbien transformé par un vecteur tel que revendiqué dans la revendication 5.

7. Procédé pour produire un polypeptide recombinant ayant une activité de facteur chimiotactique humain pour les monocytes comprenant la culture d'un hôte microbien transformé tel que revendiqué dans la revendication 6, dans des conditions qui sont appropriées à l'expression d'un polypeptide ayant une activité de facteur chimiotactique humain pour les monocytes, et la récupération dudit polypeptide.

8. Polypeptide purifié ayant une activité de facteur chimiotactique humain pour les monocytes que l'on peut obtenir par un procédé tel que revendiqué dans la revendication 7.

9. Polypeptide essentiellement pur ayant une activité de facteur chimiotactique humain pour les monocytes constitué de la séquence en acides aminés [I] décrite dans la revendication 1 ou de toute partie de ladite séquence en acides aminés capable de présenter une activité de facteur chimiotactique humain pour les monocytes.

10. Polypeptide essentiellement pur ayant une activité de facteur chimiotactique humain pour les monocytes tel que revendiqué dans la revendication 9, dans lequel les acides aminés 1 à 6 N-terminaux sont supprimés.

## Revendications (Revendications pour l'(les) Etat(s) contractant(s) suivant(s): ES)

1. Procédé d'isolement d'une séquence d'ADN isolée codant pour l'expression d'un polypeptide ayant une activité de facteur chimiotactique humain pour les monocytes comprenant la séquence en acides aminés (I) ou toute partie de ladite séquence en acides aminés capable de présenter une activité de facteur chimiotactique humain pour les monocytes 
Gln Pro Asp Ala Ile Asn Ala Pro Val Thr
Cys Cys Tyr Asn Phe Thr Asn Arg Lys Ile
Ser Val Gln Arg Leu Ala Ser Tyr Arg Arg
Ile Thr Ser Ser Lys Cys Pro Lys Glu Ala
Val Ile Phe Lys Thr Ile Val Ala Lys Glu
Ile Cys X Asp Pro Lys Gln Lys Trp Val
Gln Asp Ser Met Asp His Leu Asp Lys Gln
Thr Gln Thr Pro Lys Thr
[I]
 (dans laquelle X signifie Ala ou Thr), ledit procédé faisant usage d'une technique d'ADN recombinant.

2. Procédé selon la revendication 1, dans lequel la séquence d'ADN isolée a la séquence nucléotidique représentée par la formule A 
CAGCCAGATGCAATCAATGCCCCAGTCACC
TGCTGYTATAACTTCACCAATAGGAAGATC
TCAGTGCAGAGGCTCGCGAGCTATAGAAGA
ATCACCAGCAGCAAGTGTCCCAAAGAAGCT
GTGATCTTCAAGACCATTGTGGCCAAGGAG
ATCTGTRCTGACCCCAAGCAGAAGTGGGTT
CAGGATTCCATGGACCACCTGGACAAGCAA
ACCCAAACTCCGAAGACT
formule [A]
 (dans laquelle Y signifie C ou T, et R signifie G ou A).

3. Procédé d'isolement d'une séquence d'ADN privée des 23 à 29 codons de l'extrémité 5'-terminale d'un ADN codant pour l'expression d'un précurseur de facteur chimiotactique humain pour les monocytes comprenant la séquence en acides aminés [II] ou son ADN mutant allélique 
Met Lys Val Ser Ala Ala Leu Leu Cys Leu
Leu Leu Ile Ala Ala Thr Phe Ile Pro Gln
Gly Leu Ala Gln Pro Asp Ala Ile Asn Ala
Pro Val Thr Cys Cys Tyr Asn Phe Thr Asn
Arg Lys Ile Ser Val Gln Arg Leu Ala Ser
Tyr Arg Arg Ile Thr Ser Ser Lys Cys Pro
Lys Glu Ala Val Ile Phe Lys Thr Ile Val
Ala Lys Glu Ile Cys X Asp Pro Lys Gln
Lys Trp Val Gln Asp Ser Met Asp His Leu
Asp Lys Gln Thr Gln Thr Pro Lys Thr

[II]
 (dans laquelle X représente Ala ou Thr), ledit procédé faisant usage d'une technique d'ADN recombinant.

4. Procédé selon la revendication 3, dans lequel ledit ADN codant pour l'expression d'un précurseur de facteur chimiotactique humain pour les monocytes possède une séquence nucléotidique représentée par l'une des formules suivantes 
ATGAAAGTCTCTGCCGCCCTTCTGTGCCTG 30
MetLysValSerAlaAlaLeuLeuCysLeu (10)
CTGCTCATAGCAGCCACCTTCATTCCCCAA 60
LeuLeuIleAlaAlaThrPheIleProGln (20)
GGGCTCGCTCAGCCAGATGCAATCAATGCC 90
GlyLeuAlaGlnProAspAlaIleAsnAla (30)
CCAGTCACCTGCTGYTATAACTTCACCAAT 120
ProValThrCysCysTyrAsnPheThrAsn (40)
AGGAAGATCTCAGTGCAGAGGCTCGCGAGC 150
ArgLysIleSerValGlnArgLeuAlaSer (50)
TATAGAAGAATCACCAGCAGCAAGTGTCCC 180
TyrArgArgIleThrSerSerLysCysPro (60)
AAAGAAGCTGTGATCTTCAAGACCATTGTG 210
LysGluAlaValIlePheLysThrIleVal (70)
GCCAAGGAGATCTGTRCTGACCCCAAGCAG 240
AlaLysGluIleCys X AspProLysGln (80)
AAGTGGGTTCAGGATTCCATGGACCACCTG 270
LysTrpValGlnAspSerMetAspHisLeu (90)
GACAAGCAAACCCAAACTCCGAAGACTTGA 300
AspLysGlnThrGlnThrProLysThr*** (99)
ACACTCACTCCACAACCCAAGAATCTGCAG 330
 (dans laquelle Y est C ou T et R est G ou A).

5. Procédé de construction d'un vecteur contenant une séquence d'ADN définie dans l'une quelconque des revendications précédentes, ledit procédé faisant usage d'une technique d'ADN recombinant.

6. Procédé de production d'un hôte microbien transformé par un vecteur défini dans la revendication 5, comprenant les étapes d'introduction d'un vecteur dans un hôte microbien que l'on souhaite transformer.

7. Procédé de production d'un polypeptide recombinant ayant une activité de facteur chimiotactique humain pour les monocytes comprenant la production d'un hôte microbien transformé tel que revendiqué dans la revendication 6, la culture dudit hôte microbien dans des conditions qui sont appropriées à l'expression d'un polypeptide ayant une activité de facteur chimiotactique humain pour les monocytes, et la récupération dudit polypeptide.

8. Procédé de production d'un polypeptide purifié ayant une activité de facteur chimiotactique humain, ledit procédé faisant usage d'une technique de purification des protéines.

9. Procédé de production d'un polypeptide essentiellement pur ayant une activité de facteur chimiotactique humain pour les monocytes constitué de la séquence en acides aminés [I] décrite dans la revendication 1 ou de toute partie de ladite séquence en acides aminés capable de présenter une activité de facteur chimiotactique humain pour les monocytes, ledit procédé faisant usage d'une technique de purification des protéines.

10. Procédé de production d'un polypeptide essentiellement pur ayant une activité de facteur chimiotactique humain pour les monocytes tel que revendiqué dans la revendication 9, dans lequel les acides aminés 1 à 6 N-terminaux sont supprimés.
